# EUROPEAN PATENT APPLICATION

(11) **EP 1 516 612 A2**
(43) Date of publication of application: **23.03.2005**
(21) Application number: 04255652.2
(22) Date of filing: 17.09.2004
(51) Int. Cl.: A61K 7/032, A45D 33/00

(54) **Two-step mascara**

(30) Priority: 19.09.2003 US 666368; 16.01.2004 US 759614
(71) Applicant: l'Oreal SA, 75008 Paris (FR)
(72) Inventor: Patel, Lilavati, Edison, New Jersey 08837 (US); Sterphone, Stacy, Hillsborough, New Jersey 08844 (US)
(74) Representative: Bentham, Stephen

(57) **Abstract**

Disclosed are methods for applying mascara compositions to eyelashes, comprising applying washable mascara followed by applying waterproof mascara. Mascara products and systems, and packages containing the products are also disclosed.

## Description

### BACKGROUND OF THE INVENTION

Mascaras are commonly prepared as wax-based formulations according to two types. Washable mascaras, which can be removed with soap and water, generally come in the form of an emulsion of waxes in water such as creams, as well as in gels. Waterproof mascaras, which require use of oils for removal, generally come in the form of dispersions of waxes in organic solvents.

Each type of mascara has advantages and disadvantages. Application of washable mascaras enhances volume; that is, they thicken the eyelashes. However, they are prone to smudge such as when rubbed. In addition, they are not resistant to water. Waterproof mascaras on the other hand are relatively water-resistant and are less likely to run or smudge. However, unlike washable mascaras, they cannot be removed simply with soap and water. They require the use of organic based cleansing agents such as mineral oil.

There have been many developments in cosmetics for application to eyelashes and eyebrows aimed at improvement one or more of these properties, or other properties such as ease of application, homogeneity, comfort and good hold. For example, mixtures of waxes have been used to combine properties such as film hardness and adhesion to the lash. See, e.g., WO-A-95/15741. U.S. Patent 6,464,967 teaches the use of specific polyolefin waxes in mascara compositions. U.S. Patent 6,375,941 is directed to a wax-free mascara composition that contains film-forming polyurethane. U.S. Patent 5,879,668 teaches waterproof mascara in the form of a water-in-oil (W/O) emulsion in which water (the disperse phase) is emulsified in oil (the continuous phase). The oil is preferably a C₁₀ - C₁₄ saturated, linear or branched, hydrocarbon, more preferably a C₁₁ - C₁₃ saturated, linear or branched, hydrocarbon, more preferably a branched C₁₂ saturated hydrocarbon such as isododecane. Of course, mixtures of these hydrocarbons may be used and often are provided by commercial "technical" grades of these hydrocarbons. The emulsions are contain solid particles, preferably pigment particles and preferably at least two different types of surfactants, one being an a "oil surfactant" having a hydrophobic-lipophobic balance (HLB) of from 3-4.5, the other being a "water surfactant" having an HLB of 5.5-7.5.

### SUMMARY OF THE INVENTION

A first aspect of the present invention is directed to a method of applying mascara to eyelashes, comprising: a) applying a washable mascara composition to the eyelashes; followed by b) applying a waterproof mascara composition to the eyelashes. Eyelashes treated in accordance with the method of the present invention -- which includes real and false eyelashes -- may have enhanced thickness and exhibit greater water- and smudge-resistance, and ease of removal, compared to typical waterproof and washable mascaras when used alone.

A second aspect of the present invention is directed to a product or system for applying mascara to eyelashes, comprising a) a first reservoir that contains a washable mascara composition, b) a second reservoir that contains a waterproof mascara composition, and c) at least one applicator comprising first and second applicator members for applying the respective mascara compositions.

A third aspect of the present invention is directed to a package containing a system for applying mascara to eyelashes, comprising a) a first reservoir that contains a washable mascara composition, b) a second reservoir that contains a waterproof mascara composition, and c) at least one applicator comprising first and second applicator elements or members for applying the respective mascara compositions.

### BRIEF DESCRIPTION OF THE DRAWINGS

FIG. 1 is a schematic illustration of an embodiment of a package containing a system for applying mascara to eyelashes according to the present invention.

FIG. 2 is an exploded schematic view of the system of FIG. 1, showing schematic details of the applicators.

FIGS. 3A and 3B are schematic illustrations of alternate embodiments of a system for applying mascara to eyelashes.

FIGS. 4 and 5 are graphs showing evaluations of various application attributes following application of an embodiment of a system of the present invention to eyelashes, wherein results shown in FIG. 4 were based on an evaluation conducted immediately after application of the system, and the results shown in FIG. 5 were based on an evaluation conducted about 4 hours after application of the system.

### DETAILED DESCRIPTION

Washable mascaras can be removed simply by use of soap and water. Waterproof mascaras are not as easily removable, and require use of oil such as mineral oil. Washable or aqueous mascaras generally come in the form of an emulsion of waxes in water (sometimes referred to as cream mascaras), or gels, whereas waterproof mascaras generally come in the form of dispersions of a fatty phase that includes one or more waxes in organic solvents (which when lacking water are sometimes referred to as anhydrous mascaras), or water-in-oil emulsions (see, e.g., U.S. Patent 5,879,668). Washable mascaras and waterproof mascaras may contain many similar ingredients (e.g., waxes and film-forming polymers), the main differences between them being in the relative amounts of the ingredients, particularly water. Generally, water content of washable mascaras ranges from about 20 to about 80% by weight, and preferably from about 30 to about 60% by weight of the mascara composition. In contrast, water content of waterproof mascaras generally ranges from about 0 to about 60% by weight, and preferably from about 0 to about 35% by weight of the mascara composition. One or more water-miscible solvents may also be present in either type of mascara. Examples include lower monoalcohols containing from 1 to 5 carbon atoms, C₃ - C₄ ketones and C₃ - C₄ aldehydes. A preferred water-miscible solvent is ethanol. The content of water-miscible solvents generally ranges from about 0.1% to about 15% by weight, and preferably from about 1% to about 8% by weight relative to the total weight of the mascara composition.

Washable and waterproof mascaras usually have different viscosities. Viscosity is important from the standpoints of fast and easy application of the composition, as well as uniform coating over the entire length of the eyelashes. Generally, viscosity of washable mascaras ranges from about 10 to about 60 pascal seconds (Pa*s), and preferably from about 20 to about 40 Pa*s, whereas viscosity of waterproof mascaras ranges from about 10 to about 70 Pa*s, and preferably from about 10 to about 40 Pa*s. Viscosity is measured at 25 °C with a Rheomat RM 180 viscometer fitted with a No. 4 rotor, wherein the measurement is carried out after spinning the rotor for 10 minutes (after which time stabilization of the viscosity and of the rotor spin speed are observed), at a shear rate of 200 s⁻¹.

Viscosity may be adjusted by adding a thickener. Representative examples include cellulose-based thickeners, for example, water-soluble cellulose-based thickeners, such as hydroxyethylcellulose, methylcellulose, hydroxypropylcellulose and carboxymethylcellulose. Among these thickeners, specific examples include the gums sold under the name "Cellosize QP 4400 H" by the company Amerchol, guar gum, in particular, those sold under the name Vidogum GH 175 by the company Unipectine and under the name Jaguar C by the company Meyhall, the quaternized guar gum sold under the name "Jaguar C-13-S" by the company Meyhall, nonionic guar gums comprising C₁ -C₆ hydroxyalkyl groups, e.g., hydroxymethyl, hydroxyethyl, hydroxypropyl and hydroxybutyl groups. Such guar gums are sold, in particular, under the trade names Jaguar HP8, Jaguar HP60, Jaguar HP120 and Jaguar HP 105 by the company Meyhall, or under the name Galactasol 40H4FD2 by the company Aqualon. Other examples of thickeners include xanthan gum, carob gum, scleroglucan gum, gellan gum, rhamsan gum and karaya gum, alginates, maltodextrin, polysaccharide resins such as starch and its derivatives, hyaluronic acid and its salts, clays, and, in particular, montmorillonites, hectorites and laponites, crosslinked polyacrylic acids, such as the "Carbopol" products from the company Goodrich, the polyglyceryl (meth)acrylate polymers sold under the names "Hispagel" or "Lubragel" by the companies Hispano Quimica or Guardian, polyvinylpyrrolidone (PVP), polyvinyl alcohol, crosslinked acrylamide polymers and copolymers, such as those sold under the names "PAS 5161" or "Bozepol C" by the company Hoechst, "Sepigel 305" by the company SEPPIC, crosslinked methacryloyloxyethyltrimethylammonium chloride homopolymers sold under the name "Salcare SC95" by the company Allied Colloid, and associative polymers and, in particular, associative polyurethanes.

The mascaras suitable for use in the present invention typically contain a thickener in an effective amount for the composition to have the viscosity as defined above. The thickener content can range, for example, from 0.1% to 10% by weight relative to the total weight of the composition, and preferably from 0.5% to 5% by weight.

A variety of waxes may be present in the mascaras of the present invention, including waxes of animal origin, waxes of plant origin, waxes of mineral origin and waxes of synthetic origin. Examples of waxes of animal origin include beeswaxes, lanolin waxes and Chinese insect waxes. Examples of waxes of plant origin include rice waxes, carnauba wax, candellila wax and ouricurry wax, cork fibre waxes, sugar cane waxes, Japan waxes, sumach wax and cotton wax. Examples of waxes of mineral origin include paraffins, microcrystalline waxes, montan waxes and ozokerites. Examples of waxes of synthetic origin include polyolefin waxes, e.g., polyethylene waxes, waxes obtained by Fischer-Tropsch synthesis, waxy copolymers and their esters, and silicone waxes.

Alternatively, hydrogenated oils of animal or plant origin may be used. Examples include hydrogenated jojoba waxes and hydrogenated oils which are obtained by catalytic hydrogenation of fats composed of a C₈ - C₃₂ linear or nonlinear fatty chain, hydrogenated sunflower oil, hydrogenated castor oil, hydrogenated copra oil, hydrogenated lanolin and hydrogenated palm oils. The waxes are preferably solid and rigid at temperatures below 50 °C.

Generally, wax content of washable mascaras ranges from about 0 to about 50% by weight, and preferably from about 10 to about 45% by weight of the mascara composition. Wax content of waterproof mascaras generally ranges from about 0 to about 40% by weight, and preferably from about 5 to about 35% by weight of the mascara composition.

In some embodiments, the washable mascara composition, the waterproof mascara composition, or both, may contain fibers. The fibers useful in the present invention may be chosen from natural and synthetic fibers. Natural fibers include, but are not limited to, cotton, silk, wool, and other keratin fibers. Synthetic fibers include, but are not limited to, polyester, rayon, nylon and other polyamide fibers.

Yet other fibers useful in the present invention include those described in EP 1172078. The fibers disclosed in this publication include types of elastofibers. These fibers are chemical fibers, extremely stretchable, and which regain their primary shape as soon as the tractive force is interrupted. Representative examples include elastane (abbreviations: EL or Spandex®)), highly polymerized fibers, which contain at least 85% by weight of segmented polyurethane, and elastodiene fibers (abbreviation: ED) containing synthetic polyisoprenes or high polymers, which are obtained from the polymerization of one or more dienes, by optionally adding one or several vinyl monomers. Rubbery fibers (abbreviation: LA) issued from natural rubber may also be included in the second group. The elastodienes are often vulcanized. A fiber composed of both polyamide and polyurethane also has elastic properties.

The fibers, may, for example, be present in the washable composition, the waterproof composition, or in both compositions, and may be the same or different. The fibers are present in an amount generally ranging from about 0.1% to about 20% relative to the total weight of the composition. In some embodiments, the fibers are present in an amount ranging from about 0.2% to about 10% relative to the total weight of the composition. The fibers typically have an average length ranging from about 0.5 mm to about 4.0 mm, such as from about 1.5 mm to about 2.5 mm.

The compositions according to the invention may additionally comprise a film-forming polymer which can be solubilized and/or in the form of particles in dispersion in the aqueous phase. The film-forming polymer can be selected from keratin derivatives, such as keratin hydrolysates and sulphonic keratins; anionic, cationic, amphoteric or nonionic derivatives of chitin or chitosan; cellulose derivatives such as hydroxyethylcellulose, hydropropylcellulose, methylcellulose, ethylhydroxyethylcellulose, carboxymethylcellulose, and quaternized derivatives of cellulose; acrylic polymers or copolymers, such as polyacrylates or polymethacrylates; polyvinylpyrrolidones (PVP) and vinyl copolymers, such as methyl vinyl ether-maleic anhydride copolymers, or vinyl acetate-crotonic acid copolymer; water-dispersible anionic polyesteramide and/or polyester polymers comprising monomers bearing a functional group -- SO₃M, in which M represents a hydrogen atom, an ammonium ion NH₄⁺ or a metal ion, such as, for example, an Na⁺, Li⁺, K⁺, Mg²⁺, Ca²⁺, Cu²⁺, Fe²⁺ or Fe³⁺ ion. Specific examples of the polymers described in U.S. Patents 3,734,874, 4,233,196 and 4,304,901. Polyurethane polymers, especially anionic, cationic, nonionic or amphoteric polyurethanes, acrylic polyurethanes, polyvinylpyrrolidone polyurethanes, polyester polyurethanes, polyether polyurethanes, polyureas, polyurea/polyurethanes, and mixtures thereof; and polymers of natural origin, modified if desired, such as gum arabic, guar gum, xanthan derivatives, karaya gum; alginates and carragheenates; glycoaminoglycans, hyaluronic acid and its derivatives; shellac, sandarac gum, dammars, elemis and copals, are also useful. The film-forming polymer can be present in the mascara compositions in an amount of dry matter generally ranging from about 0.1% to about 20% by weight relative to the total weight of the mascara composition.

Preferred film-formers include combinations of a first tacky film former soluble or dispersible in water, and a second tacky film former soluble in oil. For purposes of this invention, the term "soluble or dispersible in water" means that the substance in question will not precipitate out or coagulate, *e.g.*, that it dissolves up to the limit of saturation. The term "soluble in oil" means "miscible in oil"; in other words, if a substance is not soluble in oil, it is immiscible, forming distinct layering in the oil phase, an indication that the substance is not compatible or soluble in the oil phase. For purposes of this invention, "tacky" is defined as sticky or adhesive to the touch. The combination of tacky film formers may allow, in at least some embodiments, one or more of the following to occur: allow the fibers to adhere to the eyelashes, allow optimal water resistance and allow minimal flaking.

Examples of the at least one tacky film former soluble or dispersible in water include polyvinyl alcohols (such as the AIRVOL series from Air Products); polyvinyl acetates (such as FULATEX (R) sold by H.B. Fuller Co.); cellulose acetate phthalate aqueous dispersions (such as AQUACOAT CPD sold by FMC Corp.); and acrylates copolymers, such as DAITOSOL 5080 AD sold by Kobo Products, vinylpyrrolidone/acrylates/lauryl methacrylate copolymers (such as STYLEZE 2000 sold by ISP), acrylates/C₁₋₂ succinates/hydroxyacrylates copolymers (such as ALLIANZ LT-120 sold by ISP), PVP/DMAPA acrylates copolymers (such as STYLEZE CC-10 sold by ISP), and crosslinked poly (2-ethylhexyl acrylates) in water (such as GEL-TAC 100 series sold by API). In one embodiment, the at least one tacky film former soluble or dispersible in water is chosen from an acrylates copolymer and polyvinyl acetates. The at least one tacky film former soluble or dispersible in water is present in the mascara composition in an amount of dry matter generally ranging from about 0.1% to about 20% relative to the total weight of the composition. In some embodiments, the at least one tacky film former soluble or dispersible in water is present in an amount ranging from about 1% to about 15%, relative to the total weight of the composition. In yet other embodiments, the at least one tacky film former soluble or dispersible in water is present in an amount ranging from about 1% to about 10%, relative to the total weight of the composition.

The at least one tacky film former soluble in oil is preferably chosen from hydrogenated polyisobutenes, adipic acid/diethylene glycol/glycerin crosspolymers (such as that sold as LEXOREZ 100 by Inolex), polyethylenes, and polyvinyl laurates. In some embodiments, the at least one oil-soluble tacky film former is chosen from hydrogenated polyisobutenes. Hydrogenated polyisobutenes are available from, for example, Collaborative Laboratories, East Setauket, NY, under the name POLYSYNLANE. In some embodiments, the hydrogenated polyisobutenes to be used in the claimed invention have a weight average molecular weight of greater than 1500. In other embodiments, the hydrogenated polyisobutenes have a weight average molecular weight greater than 2000 and in yet other embodiments, they have a weight average molecular weight greater than 3000. The at least one tacky film former soluble in oil may, for example, be present in the composition in an amount generally ranging from about 0.5% to about 30%, relative to the total weight of the composition. In some embodiments, the at least one tacky film former soluble in oil is present in the composition in an amount ranging from about 1% to about 20%, relative to the total weight of the composition. In yet other embodiments, the at least one tacky film former soluble in oil may be present in the composition in an amount ranging from about 1% to about 15%, relative to the total weight of the composition.

The mascara compositions of the invention may contain emulsifying surfactants. Surfactants can be selected from amphoteric, anionic or nonionic surfactants. See, e.g., *Encyclopedia of Chemical Technology, KIRK-OTHMER*, volume 22, pp. 333-432, 3rd edition, 1979, Wiley, for the definition of the properties and (emulsifying) functions of the surfactants, in particular pp. 347-377 of this publication regarding anionic and nonionic surfactants. Examples of surfactants useful in the mascara compositions of the invention are include as nonionic surfactants, fatty acids, fatty alcohols, polyethoxylated fatty alcohols or polyglycerolated fatty alcohols, such as polyethoxylated stearyl alcohols or cetylstearyl alcohols, esters of fatty acid and sucrose, and glucose alkyl esters, in particular polyoxyethylenated C₁ - C₆ alkyl glucose fatty esters, and as anionic surfactants, C₁₆ - C₃₀ fatty acids neutralized by amines, ammonia or the alkali metal salts thereof. Examples of amphoteric surfactants include betaines, sultaines, hydroxysultaines, alkyl amphodiacetates, alkyl amphodipropionates, and imidazolines, or salts thereof. Other fatty acid condensates such as those formed with amino acids, proteins, and the like are suitable as well. Specific examples include cocamphodipropionate, e.g., Miranol C2M-SF (disodium cocamphodipropionate), in its salt-free form, available from Rhone-Poulenc, and Crosultaine C-50 (cocamidopropyl hydroxysultaine), available from Croda.

In washable mascaras, surfactants are selected in order to obtain an oil-in-water emulsion. Preferred examples include triethanolamine and stearic acid. In waterproof mascaras, surfactants are often used to facilitate dispersion of pigments. Preferred examples include lecithins. Lecithins are mixtures of phospholipids, i.e., of diglycerides of fatty acids linked to an ester of phosphoric acid. Preferably, lecithins are diglycerides of stearic, palmitic, and oleic acids linked to the choline ester of phosphoric acid. Lecithin is usually defined either as pure phosphatidyl cholines or as crude mixtures of phospholipids which include phosphatidyl choline, phosphatidyl serine, phosphatidyl ethanolamine, phosphatidyl inositol, other phospholipids, and a variety of other compounds such as fatty acids, triglycerides, sterols, carbohydrates, and glycolipids. See, e.g., U.S. Patent 6,015,574 and 6,221,389. Lecithins may also be present in washable mascaras. Surfactants are generally present in amounts ranging from about 2 to about 30% by weight, and preferably from about 5% to about 15% by weight, relative to the total weight of the mascara composition.

The composition of the invention may additionally comprise ingredients that are commonly used in cosmetics, such as plasticizers, coalescence agents, fillers, dyestuffs, such as pigments or dyes, preserving agents, oils, cosmetic agents, such as moisturizers and anti-UV agents vitamins, trace elements, softeners, sequestrants, perfumes, oils, silicones, proteins, ceramides, cohesion agents, and the basifying or acidifying agents that are commonly employed in the cosmetics field.

In terms of fillers, silica is particularly useful to obtain a thicker make-up effect on the eyelashes. Starches such as rice starch, talc and polytetrafluoroethylene are highly compatible with aqueous medium and allow a smooth, shiny film of make-up to be obtained. Fillers are generally present in the mascara compositions in an amount of about 0.1% to about 6% by weight relative to the total weight of the mascara composition.

In addition to the foregoing teachings, examples of specific washable and waterproof mascara compositions are legion in the prior art. See, e.g., U.S. Patent 6,264,933 for examples of waterproof mascaras, and U.S. Patent 5,985,258 for examples of washable mascaras.

The methods of the present are practiced simply by applying to the eyelashes the washable mascara, followed by applying the waterproof mascara. The waterproof mascara may be applied immediately after application, but generally anytime from about 30 seconds to about 10 minutes after application of the washable mascara.

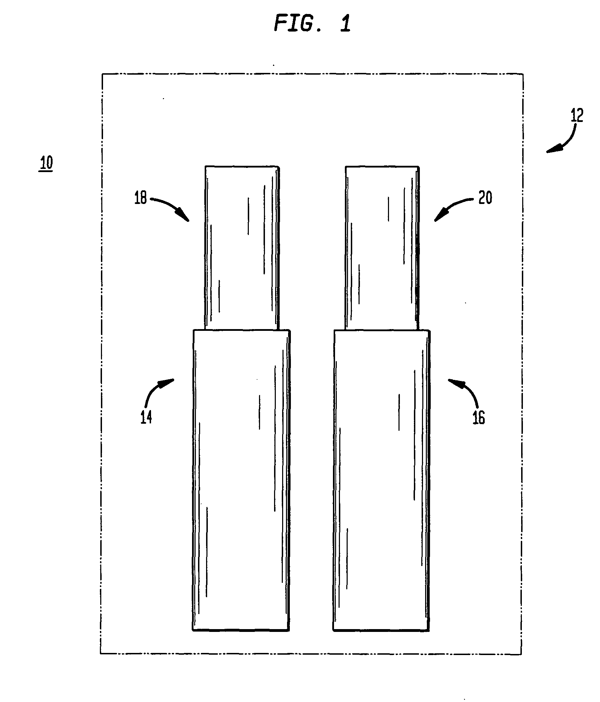

With reference to FIGS. 1-3, the mascara compositions of the invention may be provided in a mascara product or system comprising two reservoirs, each of which contains one of the mascara compositions, and applicators for applying each of the compositions to the eyelashes. This mascara product or system may be conveniently packaged in a variety of forms. As best shown in FIG. 1, one example of such preferred packaging includes a blister pack 10. The reservoirs 14 and 16 are housed in the blister pack 10, which comprises translucent plastic material (not shown) attached to a cardboard substrate backing 12. The backing may be generally rectangular in shape. Any shape that is capable of preventing any direct handling of the products by customers is contemplated and within the scope of the present invention. See, e.g., U.S. Patent 6,378,727.

Each reservoir 14 and 16 is positioned within the blister pack 10, preferably in a side-by-side manner. Each reservoir includes corresponding applicator portions 18 and 20, which are discussed below in detail.

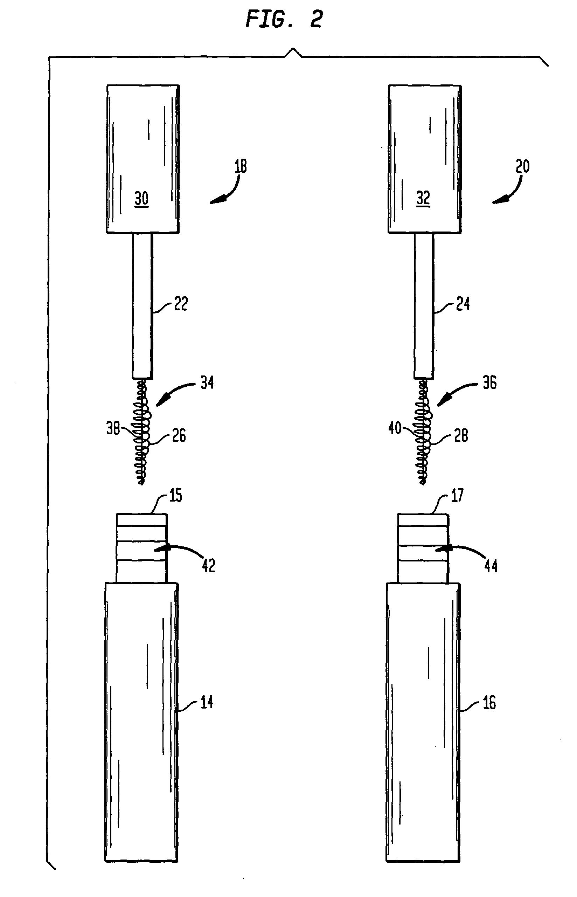

The reservoirs typically are provided with an opening 15, 17 that accommodates a liquid-removal (or applicator) system. As best seen in FIG. 2, the applicator portions 18, 20 typically comprise a rod 22, 24 equipped at a first end with an applicator member 26, 28 such as a brush, and at a second end with a handle component 30, 32 that acts as both a means for manipulating the applicator by the user, and as a cap for closing the reservoir, preferably in a leak-tight manner (e.g., the handle component itself is configured to close the reservoir).

The applicator member 26, 28 preferably comprises bristles 34, 36 arranged radially around a twisted core 38, 40, such as a metal core. The brush can be of varied shapes and can comprise cutout sections. Mascara brushes are described, for example, in French Patent Application No. FR-A-2,607,373 and European Patent Application Nos. EP-A-611,170, EP-A-811,336, EP-A-811,337 and EP-A-842,620.

The respective applicator members 26, 28 may be the same or different. In some embodiments, they are different. For example, application of the washable mascara may be facilitated with a relatively full and soft brush 34. An example of such a brush is described in U.S. Patent 4,887,622. The disclosed brushes include a central core formed by a twisted iron wire holding a helical array of radial bristles in a regular manner characterized by the fact that the bristles have a diameter between about 0.10 and about 0.25 millimeters (mm), and the number of bristles per turn being between about 10 and about 40.

A preferred example of an applicator member 28 for applying waterproof mascara is described in U.S. Patent 4,993,440. The brushes disclosed in this patent contain a central elongate core around which are implanted bristles disposed in a substantially radial fashion and regularly distributed. Each bristle 36 of at least one portion of the brush comprises on its surface at least one capillary channel (not shown) extending substantially from its base as far as its tip. Preferably, all the bristles of the brush have capillary channels. The brush is evenly charged and a homogeneous distribution of the mascara on the eyelashes is achieved. Further, the systems and packages of the present invention may further contain indicia such as directions or instructions (e.g., printed information) for using the mascara compositions.

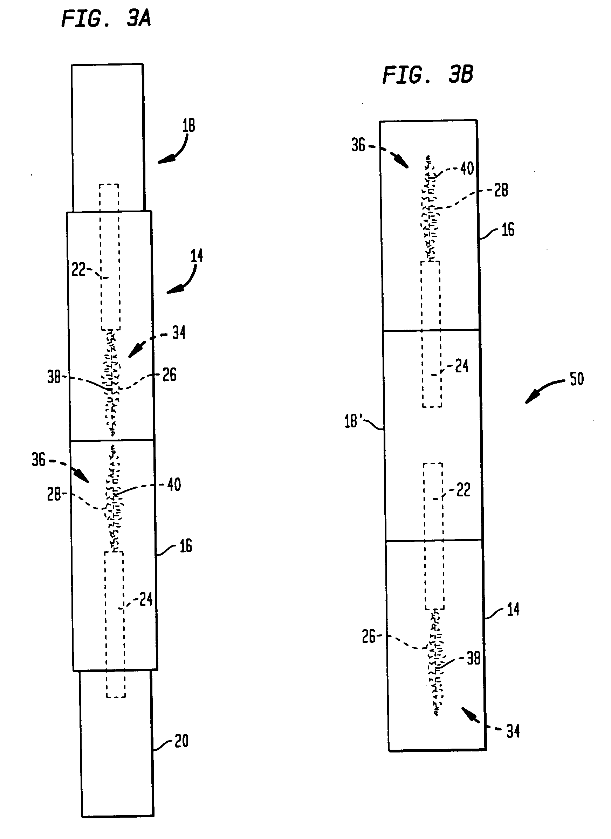

Alternate embodiments of mascara systems are shown in FIGS. 3A and 3B. In FIG. 3A, the reservoirs 14 and 16 are integral with each other, e.g., their respective bottom or side surfaces are fastened together in some manner. In another alternate embodiment shown in Fig. 3B, one applicator 50 is provided, which contains a single handle component 18' and two stems 22, 24 oppositely opposed from the handle component, wherein each stem has an applicator member 26, 28 at its opposite end. The handling component contains a cap configured to close each of the reservoirs, preferably in a leak-tight manner.

The present invention is further described in terms of the following non-limiting examples. Unless otherwise indicated, all parts and percentages are on a weight-by-weight basis.

### Example 1: Washable Mascara

The following mascara composition was made with a combination of lecithin, an amphoteric surfactant and a nonionic surfactant (LAN).

| **Phase** | **CTFA Name** | |
|---|---|---|
| **A** | Water | **35.830** |
| | PVP-K-30 (PVP/VA Copolymer, GAF/BASF) | **1.000** |
| | Butylene Glycol | **2.000** |
| | Hydroxyethyl Cellulose | **0.350** |
| | Methylparaben | **0.250** |
| | Disodium EDTA | **0.200** |
| | Triethanolamine | **1.500** |
| | Simethicone (Mirasil SM, Rhodia Chimie) | **0.100** |
| | KAMA KM 13 (Polysaccharide Resin, KAMA International) | **0.950** |
| | Black Iron Oxide | **8.000** |
| | Polymethyl methacrylate isopropyl titanium triisostearate | **2.000** |
| | Nylon 12 (Orgasol 2002D, Atochem) | **2.000** |
| | | |
| **B** | Beeswax | **4.700** |
| | Glyceryl Stearate | **4.000** |
| | Paraffin | **2.700** |
| | Carnauba | **3.400** |
| | Stearic Acid | **3.000** |
| | Butylparaben | **0.100** |
| | PVP Eicosene Copolymer (GANEX V220, ISP) | **1.500** |
| | Synthetic Wax (PERFORMA V103, New Phase) | **1.000** |
| | 2-Oleamido-1, 3- Octadecanediol (Ceramide) | **0.020** |
| | | |
| **C** | Cyclopentasiloxane (DC 245, Dow Corning) | **2.000** |
| | Cyclopentasiloxane /Dimethiconol (DC 2-9071, Dow Corning) | **3.000** |
| | Silica | **1.000** |
| | Polyethylene (MICROPOLY 524, Presperse) | **2.000** |
| | | |
| **D** | Lecithin (ALCOLEC F100 (L) Disodium Cocoamphodipropionate (MIRANOL) (A) Isoceteth-20 (ARLASOLVE 200) (N) | **0.100, 0.400 and 0.500, respectively, totaling 1.000** |
| | Water | **3.000** |
| | | |
| **E** | PPG-17/IPDI/DMPA/Copolymer (AVALURE UR450, BF Goodrich) | **10.000** |
| | | |
| **F** | Diazodinyl Urea | **0.300** |
| | Water | **1.000** |
| | | |
| **G** | Panthenol | **0.500** |
| | Water | **0.500** |
| **H** | Vitamin E Acetate | **0.100** |
| | | **100.000** |

To prepare the washable mascara, the PVP/VA was added to water, using a homogenizer to disperse and heat to 60°C. Premixed butylene glycol and hydroxyethylecellulose were added to the dispersion. Upon dispersion, methylparaben, disodium EDTA, triethanolamine, simethicone, and polysaccharide resin respectively, were added. Upon achieving a uniform dispersion, iron oxide was added. Polymethyl methacrylate isopropyl titanium triisostearate and Nylon -12 were added sequentially with milling until the mixture was well dispersed. The dispersion was heated to 85°C to 90°C. Phase B ingredients were mixed separately and then heated to 85°C to 90°C. Using a homogenizer, phase B was added to phase A, followed by emulsifying for 15minutes. After emulsification was complete, the mixture was transferred to sweep mixing and air-cooled to 60°C. Premixed phase C was added slowly. Once uniform, premade -premixed phase D was added slowly at 55°C.

To prepare phase D, disodium cocoamphodipropionate, isoceteth -20 and simethicone were heated to 70°C to 75°C. Once uniform, Lecithin was added while mixing at high speed using propeller mixer, until dispersed. Water was added at 70°C to 75°C while maintaining temperature at 70°C to 75°C, and mixing until uniform (which took approximately 10 minutes). Methylparaben, propylparaben and disodium EDTA were added, and once dispersed, octylacrylamide/acrylates/butylaminoethyl methacrylate copolymer was added slowly to avoid lumping. These ingredients were mixed approximately 2 hours, while maintaining the temperature at 70°C to 75°C, and then were cooled to 45°C.

Once uniform, phase E was added at 45°C slowly enough to avoid coagulation, followed by mixing until uniform. Premixed phases F, G, and H were added at 40°C, followed by cooling to 30°C to 32°C.

### Example 2: Waterproof Mascara

| | **CTFA Name** | **%** |
|---|---|---|
| **A** | Petroleum Distillates (Shellsol OMS, Shell) | **47.030** |
| | Iron Oxide | **6.500** |
| | Lecithin (Alcolec BS, American Lecithin) | **0.100** |
| | Methylparaben | **0.400** |
| | | |
| **B** | Quaternium - 18 Hectorite | **6.250** |
| | | |
| **C** | Rice Starch (Remy DRI, Remy) | **1.000** |
| | | |
| **D** | Isoparaffin (Isopar E, Exxonmobil Chem.) | **10.500** |
| | | |
| **E** | Propylene Carbonate | **0.950** |
| | | |
| **F** | Paraffin | **2.300** |
| | Carnauba | **4.700** |
| | Beeswax | **4.900** |
| | Synthetic Beeswax (Cylochem 326A, Goldschmidt (Degussa)) | **3.400** |
| | AllylStearate/VA Copolymer | |
| | (Mexomere PQ, Chimex) | **2.500** |
| | Polyvinyl Laurate(Mexomere PP, Chimex) | **1.300** |
| | Silica (Spheron P1500, Catalyst & Chemicals) | **1.000** |
| | 2-Oleamido-1, 3- Octadecanediol (Ceramide) | **0.020** |
| | | |
| **G** | Propylene Carbonate | **1.100** |
| | | |
| **H** | KAMA KM 13 (Polysaccharide Resin, KAMA International) | **1.000** |
| | Vitamin E Acetate | **0.050** |
| | | |
| **I** | Lecithin (ALCOLEC F100) (L) | **0.050** |
| | Disodium Cocoamphodipropionate (MIRANOL) (A) | **0.200** |
| | Isoceteth-20 (ARLASOLVE 200)(N) | **0.250** |
| | Simethicone (Mirasil SM from Rhodia Chimie) | **0.010** |
| | Methylparaben | **0.001** |
| | Propylparaben | **0.001** |
| | Disodium EDTA | **0.001** |
| | Octylacrylamide/Acrylates/ Butylaminoethyl Methacrylate Copolymer | **0.060** |
| | (AMPHOMER LV-71) | **0.003** |
| | Diazodinyl Urea Vitamin E Acetate | **0.005** |
| | Water | **3.419** |
| | | |
| **J** | Panthenol | **0.500** |
| | Water | **0.500** |
| | | **100.000** |

To prepare the waterproof mascara, phase A ingredients were added together and homogenized until dispersed at room temperature. Phase B ingredients were added, and homogenized until uniform, followed by addition of phase C and homogenized until dispersed. Once uniformly dispersed, phase D and E ingredients were added (at this point batch will become very heavy). The resultant dispersion was heated to 65°C to 70°C. Phase F was prepared and heated to 90°C to 95°C, and then added to the dispersion, followed by the addition of phase G. Homogenization was continued for 30 minutes, while maintaining the temperature at 65°C to 70°C. Phase H was prepared and added to the dispersion, and then homogenized until uniform. Pre-made phase I was added; the dispersion was cooled to 45°C using sweep mixing; and pre-mixed phase J was added, followed by cooling to 30°C to 32°C.

Phase I was prepared by heating disodium cocoamphodipropionate, isoceteth -20 and simethicone to 70°C to 75°C. Once uniform, lecithin was added while mixing at high speed using propeller mixer, until dispersed. Water was added at 70°C to 75°C while maintaining temperature at 70°C to 75°C, and mixing until uniform (which took approximately 10 minutes). Methylparaben, propylparaben and disodium EDTA were added, and once dispersed, octylacrylamide/acrylates/butylaminoethyl methacrylate copolymer was added slowly to avoid lumping. These ingredients were mixed approximately 2 hours, while maintaining the temperature at 70°C to 75°C, and then were cooled to 45°C.

### Example 3: Evaluation of Performance

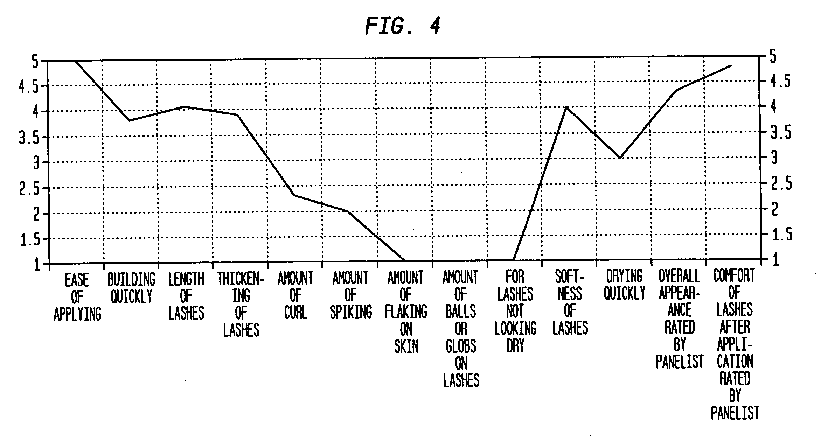

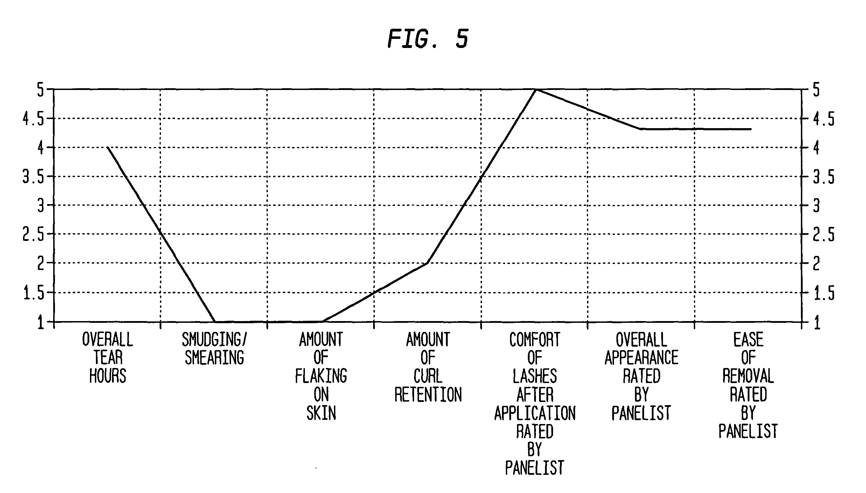

A panel study was conducted in which the washable mascara described in example 1 was applied to the eyelashes of (one/two eye/eyes) of 6 panelists, followed by the application of the waterproof mascara described in example 2. Many application attributes were evaluated, including overall wear (judged in terms of hours), and smudging/smearing, ease of application, speed of build-up, length of lashes, thickening of lashes, amount of curl, amount of spiking, amount of flaking, homogeneity of application (i.e., amount of balls or globs on lashes), softness, wetness (or lack of dryness) of appearance, time for drying, comfort, overall appearance and ease of removal. The results shown in FIG. 4 were on the basis of an evaluation conducted immediately after application. The results shown in FIG. 5 were on the basis of an evaluation conducted 4 hours after application. They show that the mascara system of the present invention was judged very favorably from all of these application attributes.

### Example 4: Washable Mascara with Fibers

The following mascara composition was made with a combination of lecithin, an amphoteric surfactant and a nonionic surfactant (LAN).

| **Washable Mascara With Fibers** | | |
|---|---|---|
| | **CTFA Name** | **%** |
| **A** | Water | **34.830** |
| | PVP-K-30 (PVP/VA Copolymer, GAF/BASF) | **1.000** |
| | Butylene Glycol | **2.000** |
| | Hydroxyethylcellulose | **0.350** |
| | Methylparaben | **0.250** |
| | Disodium EDTA | **0.200** |
| | Triethanolamine | **1.500** |
| | Simethicone (Mirasil SM from Rhodia Chimie) | **0.100** |
| | KAMA KM 13 (Polysaccharide Resin from Kama Int'l) | **0.950** |
| | Black Iron Oxide | **8.000** |
| | Polymethyl methacrylate isopropyl titanium triisostearate | **2.000** |
| | Nylon-12 (Orgasol 2002D from Atochem) | **2.000** |
| | | |
| **B** | Rayon (Rayon Flock Rcise N0003 M04) | **0.500** |
| | Poly Toluylene Co Trimellic Amide Imide (from Kermel) | **0.500** |
| | | |
| **C** | Beeswax | **4.700** |
| | Glyceryl Stearate | **4.000** |
| | Paraffin | **2.700** |
| | Carnauba wax | **3.400** |
| | Stearic acid | **3.000** |
| | Butylparaben | **0.100** |
| | PVP/eicosene Copolymer (GANEX V220, from ISP) | **1.500** |
| | PERFORMA V103 polymer (Synthetic wax from New Phase) | **1.000** |
| | 2-Oleamido-1, 3-Octadecanediol (Ceramide) | **0.020** |
| **D** | Cyclopentasiloxane (DC 245from Dow Corning) | **2.000** |
| | Cyclopentasiloxane /Dimethiconol (DC 2- 9071 from Dow Corning) | **3.000** |
| | Silica | **1.000** |
| | Polyethylene (MICROPOLY 524 from Presperse) | **2.000** |
| **E** | Lecithin (ALCOLEC F100) (L) | **0.100** |
| | Disodium Cocoamphodipropionate (MIRANOL) (A) | **0.400** |
| | Isoceteth-20 (ARLASOLVE 200) (N) | **0.500** |
| | Simethicone (Mirasil SM from Rhodia | |
| | Chimie) | **0.020** |
| | Methylparaben | **0.002** |
| | Propylparaben | **0.002** |
| | Disodium EDTA | **0.002** |
| | Octylacrylamide/Acrylates/Butylaminoethy | |
| | Methacrylate Copolymer (AMPHOMER LV-71) | **0.120** |
| | Diazodinyl Urea | **0.006** |
| | Vitamin E Acetate | **0.010** |
| | Water | **3.838** |
| **F** | PPG-17/IPDI/DMPA/Copolymer (AVALURE UR450 from BFGoodrich) z | **10.000** |
| **G** | Diazodinyl Urea | **0.300** |
| | Water | **1.000** |
| **H** | Panthenol | **0.500** |
| | Water | **0.500** |
| **I** | Vitamin E Acetate | **0.100** |
| | | **100.000** |

To prepare the washable mascara, the PVP/VA was added to water, using a homogenizer to disperse and heat to 60°C. Premixed butylene glycol and hydroxyethylcellulose were added to the dispersion. Upon dispersion, methylparaben, disodium EDTA, triethanolamine, simethicone, and polysaccharide resin respectively, were added. Upon achieving a uniform dispersion, iron oxide was added. Polymethyl methacrylate isopropyl titanium triisostearate and Nylon -12 were added sequentially with milling until the mixture was well dispersed. The resultant dispersion was transferred to a paddle mixer. The fibers of phase B were added and mixed for 15 minutes. Phases A and B were heated to 85°C to 90°C. Phase C ingredients were mixed separately and then heated to 85°C to 90°C. Phase C was added to phases A and B, followed by emulsifying for 15 minutes. After emulsification was complete, the mixture was transferred to sweep mixing and air-cooled to 60°C. Premixed phase D was added slowly. Once uniform, premade-premixed phase E was added slowly at 55°C. Once uniform, phase F was added very slowly, at a temperature of 45°C, and mixed until uniform. Premixed phases G, H and I were added at 40°C, and the resultant mixture was cooled to 30-32°C.

### Example 5: Waterproof Mascara with Fibers

| **Mascara (WATERPROOF) With Fibers** | | |
|---|---|---|
| | **CTFA Name** | **%** |
| **A** | Petroleum Distillates (Shellsol OMS from Shell) | **46.830** |
| | Iron Oxide | **6.500** |
| | Lecithin (Alcolec BS from American Lecithin) | **0.100** |
| | Methylparaben | **0.400** |
| | | |
| **B** | Quaternium - 18 Hectorite | **6.250** |
| | | |
| **C** | Rice Starch (RemyDRI from Remy) | **1.000** |
| | | |
| **D** | Isoparaffin (Isopar E from Exxonmobil Chemical) | **10.500** |
| | | |
| **E** | Propylene Carbonate | **0.950** |
| | | |
| **F** | Rayon (Rayon Flock Rcise N0003 M04) | **0.100** |
| | Poly Toluylene Co Trimellic Amide Imide (from Kermel) | **0.100** |
| | | |
| **G** | Paraffin | **2.300** |
| | Carnauba | **4.700** |
| | Beeswax | **4.900** |
| | Synthetic Beeswax (Cylochem 326A from Goldschmidt(Degussa) | **3.400** |
| | Allylstearate/VA Copolymer (Mexomere PQ from Chimex) | **2.500** |
| | Polyvinyl Laurate (Mexomere PP from Chimex) | **1.300** |
| | Silica (Spheron P1500 from catalyst & chemicals) | **1.000** |
| | 2-Oleamido-1, 3-Octadecanediol (Ceramide) | **0.020** |
| | | |
| **H** | Propylene Carbonate | **1.100** |
| | | |
| **I** | KAMA KM 13 (Polysaccharide Resin from Kama Int'l) | **1.000** |
| | Vitamin E Acetate | **0.050** |
| | | |
| **J** | Lecithin (ALCOLEC F100)(L) | **0.050** |
| | Disodium Cocoamphodipropionate (MIRANOL) | |
| | (A) | **0.200** |
| | Isoceteth-20 (ARLASOLVE 200) | |
| | (N) | **0.250** |
| | Simethicone (Mirasil SM from Rhodia | |
| | Chimie) | **0.010** |
| | Methylparaben | **0.001** |
| | Propylparaben | **0.001** |
| | Disodium EDTA | **0.001** |
| | | |
| | Octylacrylamide/Acrylates/Butylaminoethy Methacrylate Copolymer (AMPHOMER LV-71) | **0.060** |
| | Diazodinyl Urea | **0.003** |
| | Vitamin E Acetate | **0.005** |
| | Water | **3.419** |
| | | |
| **K** | Panthenol | **0.500** |
| | Water | **0.500** |
| | | **100.000** |

To prepare the waterproof mascara, phase A ingredients were added together and homogenized until dispersed at room temperature. Phase B ingredients were added, and homogenized until uniform, followed by addition of phase C and homogenized until dispersed. Once uniformly dispersed, phases D and E ingredients were added (at this point batch will become very heavy). The resultant dispersion was switched to a paddle mixer and heated to 65°C to 70°C. Phase F was added to the dispersion, followed by mixing for 15 minutes. Phase G ingredients were heated to 90-95°C, and then added to the mixture, followed by the addition of phase H. Mixing was continued for 30 minutes, while maintaining the temperature at 65°C to 70°C. Phase I was prepared and added to the dispersion, and then homogenized until uniform. Pre-made phase J was added; the dispersion was cooled to 45°C using sweep mixing; and pre-mixed phase K was added, followed by cooling to 30°C to 32°C.

All publications cited in the specification are indicative of the level of skill of those skilled in the art to which this invention pertains. All these publications are herein incorporated by reference to the same extent as if each individual publication were specifically and individually indicated to be incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A method of applying mascara to eyelashes, comprising:
a) applying a washable mascara composition to eyelashes; followed by
b) applying a waterproof mascara composition to the eyelashes.

2. The method of claim 1, wherein the eyelashes are real eyelashes.

3. The method of claim 1, wherein the eyelashes are false eyelashes.

4. The method of claim 1, wherein the washable mascara composition, the waterproof mascara composition, or both, comprises a lecithin, an amphoteric surfactant and a nonionic surfactant.

5. The method of claim 1, wherein the washable mascara composition, the waterproof mascara composition, or both contain fibers.

6. The method of claim 5, wherein the washable mascara composition contains the fibers.

7. The method of claim 5, wherein the waterproof mascara composition contains the fibers.

8. The method of claim 5, wherein both the washable and the waterproof mascara composition contain fibers, wherein the fibers in each of the composition may be the same or different.

9. The method of claim 5, wherein the fibers comprise natural fibers.

10. The method of claim 9, wherein the natural fibers comprise cotton, silk or wool fibers.

11. The method of claim 9, wherein the fibers comprise synthetic fibers.

12. The method of claim 11, wherein the synthetic fibers comprise elastomeric fibers.

13. The method of claim 11, wherein the synthetic fibers comprise polyester fibers.

14. The method of claim 11, wherein the synthetic fibers comprise polyamide fibers.

15. The method of claim 11, wherein the synthetic fibers comprise rayon fibers.

16. The method of claim 11, wherein the synthetic fibers comprise nylon fibers.

17. The method of claim 5, wherein the fibers have an average length of about 0.5mm to about 4.0mm.

18. The method of claim 17, wherein the average length is about 1.5mm to about 2.5mm.

19. The method of claim 5, wherein the mascara composition that comprises the fibers also comprises a first tacky film former soluble or dispersible in water, and a second tacky film former soluble in oil.

20. The method of claim 19, wherein the first tacky film former comprises a polyvinyl alcohol, polyvinyl acetate, vinylpyrrolidone/acrylates/lauryl methacrylate copolymer, acrylates/C1-2 succinates/hydroxyacrylates copolymer, PVP/DMAPA acrylates copolymer, cellulose acetate phthalate aqueous dispersion, or a crosslinked poly (2-ethylhexyl acrylate).

21. The method of claim 19, wherein the second tacky film former comprises a hydrogenated isobutene, an adipic acid/diethylene glycol/glycerin crosspolymer, a polyethylene or a polyvinyl laurate.

22. The method of claim 21, wherein the second tacky film former comprises a hydrogenated isobutene.

23. A mascara product or system, comprising
a) a first reservoir that contains a washable mascara composition;
b) a second reservoir that contains a waterproof mascara composition; and
c) at least one applicator comprising first and second applicator members for applying the respective mascara compositions.

24. The mascara product of claim 23, wherein said first and second reservoirs are integral with each other.

25. The mascara product of claim 23, comprising two applicators, wherein a first applicator comprises said first applicator member, and a second applicator comprising said second applicator member.

26. The mascara product of claim 23, comprising one applicator comprising a handle component for manipulation by a user and for closing said reservoirs.

27. The mascara product of claim 23, wherein the first and second applicator members are brushes.

28. The mascara product of claim 23, wherein said first and second applicator members are different.

29. The mascara product of claim 23, further comprising directions for using said washable and waterproof mascara compositions.

30. The mascara product of claim 23, wherein the washable mascara composition, the waterproof mascara composition, or both, comprises a lecithin, an amphoteric surfactant and a nonionic surfactant.

31. The mascara of claim 23, wherein the washable mascara composition, the waterproof mascara composition, or both contain fibers.

32. A package comprising a mascara product or system, comprising
a) a first reservoir that contains a washable mascara composition;
b) a second reservoir that contains a waterproof mascara composition; and
c) at least one applicator comprising first and second applicator members for applying the respective mascara compositions.

33. The package of claim 32, which is in the form of a blister pack.

34. The package of claim 32, wherein said first and second reservoirs are integral with each other.

35. The package of claim 32, comprising two applicators, wherein a first applicator comprises said first applicator member, and a second applicator comprising said second applicator member.

36. The package of claim 32, comprising one applicator comprising a handle component for manipulation by a user and for closing said reservoirs.

37. The package of claim 32, wherein the first and second applicator members are brushes.

38. The package of claim 32, wherein said first and second applicator members are different.

39. The package of claim 32, further comprising directions for using said washable and waterproof mascara compositions.

40. The package of claim 32, wherein the washable mascara composition, the waterproof mascara composition, or both, comprises a lecithin, an amphoteric surfactant and a nonionic surfactant.

41. The package of claim 32, wherein the washable mascara composition, the waterproof mascara composition, or both contain fibers.
